(19) Europäisches Patentamt
European Patent Office
Office européen des brevets

(11) Veröffentlichungsnummer: **0 484 908 A2**

(12) **EUROPÄISCHE PATENTANMELDUNG**

(21) Anmeldenummer: **91118918.1**

(22) Anmeldetag: **06.11.91**

(51) Int. Cl.⁵: **C12P 17/12**, C12N 1/20, C07D 241/18, //(C12N1/20, C12R1:01)

(30) Priorität: **08.11.90 CH 3550/90**

(43) Veröffentlichungstag der Anmeldung: **13.05.92 Patentblatt 92/20**

(84) Benannte Vertragsstaaten: **AT BE CH DE DK ES FR GB IT LI NL SE**

(71) Anmelder: **LONZA A.G.**
**Gampel**
**Wallis(CH)**

(72) Erfinder: **Kiener, Andreas, Dr.**
**Meisenweg 5**
**Visp (Kanton Wallis)(CH)**
Erfinder: **Heinzmann, Klaus**
**Wierastrasse**
**Visperterminen (Kanton Wallis)(CH)**
Erfinder: **Bokel, Michael, Dr.**
**Bäretstrasse 4**
**Visp (Kanton Wallis)(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

(54) **Mikrobiologisches Verfahren zur Herstellung von hydroxylierten Pyrazinderivaten.**

(57) Beschrieben werden Mikroorganismen, die befähigt sind, mit Pyrazin als einziger Kohlenstoff-, Stickstoff- und Energiequelle zu wachsen. Diese Mikroorganismen hydroxylieren Pyrazinderivate der allgemeinen Formel

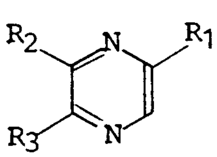

I

zu hydroxylierten Pyrazinderivaten der allgemeinen Formel

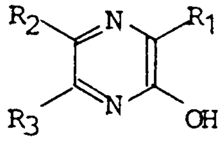

II

wobei letztere im Wachstumsmedium akkumuliert werden.

EP 0 484 908 A2

Die Erfindung betrifft neue Mikroorganismen, die mit Pyrazin wachsen, und Pyrazinderivate der allgemeinen Formel

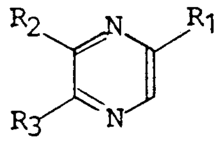

I

worin $R_1$ ein Wasserstoffatom oder ein Halogenatom bedeutet und $R_2$ und $R_3$ gleich oder verschieden sind und ein Wasserstoffatom oder eine $C_1$-$C_4$-Alkylgruppe, bedeuten, aber $R_1$, $R_2$ und $R_3$ nicht gleichzeitig Wasserstoff sind, hydroxylieren, sowie ein Verfahren zur Herstellung von hydroxylierten Pyrazinderivaten.

Hydroxylierte Pyrazinderivate sind beispielsweise wichtige Zwischenprodukte zur Herstellung von Methoxyalkylpyrazinen. Methoxyalkylpyrazine sind wesentliche Bestandteile von Aromastoffen (Maga und Sizer, J.Agric.Food Chem., 21, 1973, S.22-30).

Bisher sind nur chemische Verfahren zur Herstellung von hydroxylierten Pyrazinen bekannt, wie beispielsweise das von Karmas und Spoerri in J.Am. Chem.Soc., 74, 1952, S.1580-1584 beschriebene, in welchem beispielsweise 2-Hydroxy-5-methylpyrazin ausgehend von Methylglyoxal und Glycinamidhydrochlorid synthetisiert wird. Dieses Verfahren hat jedoch den Nachteil, dass das Produkt stark verunreinigt ist.

Desweiteren sind Untersuchungen über den biologischen Abbau von 2-Hydroxypyrazin in Matley und Harle, Biochem.Soc.Trans, 4, 1976, S.492-493 beschrieben.
Ein biotechnologisches Verfahren zur Herstellung von regiospezifisch hydroxylierten Pyrazinderivaten, ausgehend von substituierten Pyrazinderivaten mit Mikroorgansimen, die mit Pyrazin wachsen, ist nicht bekannt.

Der Erfindung liegt die Aufgabe zugrunde, neue Mikroorganismen zu finden, die ökonomisch auf biotechnologischem Weg und auf einfache Weise Pyrazinderivate der allgemeinen Formel I regiospezifisch hydroxylieren, sowie ein biotechnologisches Verfahren zur Herstellung von hydroxylierten Pyrazinderivaten zu entwickeln.

Diese Aufgabe wurde mit den Mikroorganismen gemäss Patentanspruch 1 und mit einem Verfahren zur Herstellung von hydroxylierten Pyrazinderivaten, gemäss Patentanspruch 5, gelöst. Diese Mikroorganismen sind befähigt,mit Pyrazin als einziger Kohlenstoff-, Stickstoff- und Energiequelle zu wachsen, und als Substrat Pyrazinderivate der allgemeinen Formel

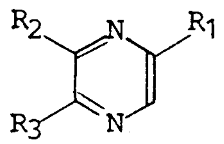

I

worin $R_1$ ein Wasserstoffatom oder ein Halogenatom bedeutet und $R_2$ und $R_3$ gleich oder verschieden sind und ein Wasserstoffatom oder eine $C_1$-$C_4$-Alkylgruppe bedeuten aber $R_1$, $R_2$ und $R_3$ nicht gleichzeitig Wasserstoff sind, in ein hydroxyliertes Pyrazinderivat der allgemeinen Formel

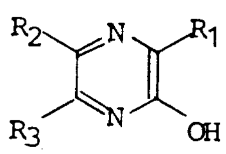

II

worin $R_1$, $R_2$ und $R_3$ die oben genannte Bedeutung haben, umzusetzen, wobei letzteres im Wachstumsmedium akkumuliert wird. Die erfindungsgemäß eingesetzten Pyrazinderivate I sind käuflich.

Erfindungsgemäss kommen alle Mikroorganismen in Frage, die Pyrazin als einzige Kohlenstoff-, Stickstoff- und Energiequelle verwerten und nach üblichen mikrobiologischen Techniken, z.B. aus Bodenproben, Kläranlagen, Erdreich, Ameisenhaufen und Komposthaufen, selektioniert werden.

Zweckmässig können alle gram-positiven und gram-negativen Mikroorganismen angewendet werden, die Pyrazin abbauen und ein Pyrazinderivat der allgemeinem Formel I als Substrat zu einem hydroxylierten Pyrazinderivat der allgemeinen Formel II hydroxylieren und dieses im Wachstumsmedium akkumulieren.

Ein bevorzugter Mikroorganismus ist Agrobacterium radiobacter DRS 3 mit der DSM-Nr. 6136, der im folgenden, aufgrund detaillierter Identifizierungsdaten, als Mikroorganismus Agrobacterium sp. (DSM-Nr. 6136) bezeichnet wird.
Dieser Stamm wurde am 7.9.1990 bei der Deutschen Sammlung von Mikroorganismen (DSM) und Zellkulturen GmbH, Mascheroder Weg lb, 3300 Braunschweig/BRD, hinterlegt.

Wissenschaftliche Beschreibung von Agrobacterium sp. (DSM-Nr. 6136)

| | | | |
|---|---|---|---|
| Zellform | Stäbchen | ADH | - |
| Breite µm | 0,6-0,8 | | |
| Länge µm | 1,5-3,0 | LDC | - |
| Beweglichkeit | + | ODC | - |
| Gram-Reaktion | - | ONPG | + |
| Lyse durch 3% KOH | + | | |
| Aminopeptidase (Cerny) | + | VP | - |
| Sporen | - | Indol | - |
| Oxidase | + | $NO_2$ aus $NO_3$ | + |
| Catalase | W * | Dentifrikation | + |
| Wachstum | | Phenylalanindesaminase | - |
| anaerob | - | | |
| 37/41°C | -/- | Lecithinase | - |
| pH 5,6 | - | | |
| Mac-Conkey-Agar | + | Urease | + |
| SS-Agar | - | | |
| Cetrimid-Agar | - | Simmons Citrat | - |
| 2% NaCl | - | | |
| | | Malonat | - |
| Pigmente | - | | |
| nicht diffundierend | - | Ketolactose | - |
| diffundierend | - | | |
| fluoreszierend | - | Hydrolyse von | |
| Pyocyanin | - | Stärke | - |
| | | Gelatine | - |
| Säure aus (OF-Test) | | Casein | - |
| Glucose aerob | - | DNA | - |
| Glucose anaerob | - | Tween 80 | - |
| | | Äsculin | + |
| Gas aus Glucose | - | | |
| | | Tyrosin-Abbau | - |
| Säure aus (ASS) ** | | | |
| Glucose | + | Alkalisierung von | |
| Fructose | + | Lackmus Milch | + |

* W = wenig ** ASS = Acetylsalicylsäure

| | | | |
|---|---|---|---|
| Xylose | + | | |
| Ethanol | + | Wuchsstoffbedarf | - |
| m-Erylthritol | + | | |
| Melezitose | - | Substratverwertung | |
| Arabinose | + | Acetat | + |
| Saccharose | + | Adipat | - |
| Cellobiose | + | Caprat | - |
| Trehalose | + | Citrat | - |
| Rhamnose | + | Glycolat | + |
| Dulcit | - | Lactat | + |
| Sorbit | + | Lävulinat | - |
| Glycerin | + | Malat | + |
| Malonat | - | | |
| Phenylacetat | - | | |
| Suberat | - | | |
| Sebacinat | - | | |
| m-Tartrat | - | | |
| L-Arabinose | + | | |
| Fructose | + | | |
| Glucose | + | | |
| Mannose | + | | |
| Maltose | + | | |
| Xylose | + | | |
| Fucose | - | | |
| Mannit | + | | |
| 2-Ketogluconat | - | | |
| N-Acetylglucosamin | + | | |
| L-Asparat | + | | |
| L-Serin | + | | |
| L-Glutamat | + | | |
| L-Histidin | - | | |
| Hydroxybutyrat | - | | |
| Betain | + | | |
| Methylamin | - | | |
| Methanol | - | | |
| Ethanol | - | | |

Hauptchinonkomponente: Ubichinon 10

Für das Verfahren zur Herstellung von hydroxylierten Pyrazinderivaten wird mit den Mikroorganismen ein Pyrazinderivat der allgemeinen Formel

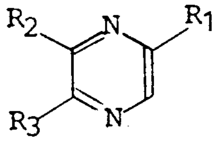

I

worin $R_1$ ein Wasserstoffatom oder ein Halogenatom bedeutet und $R_2$ und $R_3$ gleich oder verschieden sind und ein Wasserstoffatom oder eine $C_1$-$C_4$-Alkylgruppe bedeuten, aber $R_1$, $R_2$ und $R_3$ nicht gleichzeitig Wasserstoff sind, in ein hydroxyliertes Pyrazinderivat der allgemeinen Formel

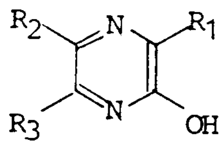

II

worin $R_1$, $R_2$ und $R_3$ die oben genannte Bedeutung haben, überführt und das angereicherte Produkt isoliert.

Vorzugsweise werden durch diese Mikroorganismen hydroxylierte Pyrazinderivate hergestellt, worin $R_1$ ein Wasserstoffatom oder ein Chloratom und $R_2$ und $R_3$ gleich oder verschieden sind und ein Wasserstoffatom, eine Methylgruppe oder Ethylgruppe, aber $R_1$, $R_2$ und $R_3$ nicht gleichzeitig Wasserstoff bedeuten.

Ebenso konnte durch diese Mikroorganismen ein neues hydroxyliertes Pyrazinderivat hergestellt werden, das 6-Ethyl-2-hydroxypyrazin.

Üblicherweise werden die Mikroorganismen vor dem eigentlichen Verfahren (Substratumsetzung) in einem Medium, ein Wachstumssubstrat enthaltend, kultiviert.

Das Wachstumssubstrat Pyrazin wird in einer Menge von 0,001 bis 10 Gew.%, bezogen auf das Kulturmedium, vorzugsweise in einer Menge von 0,001 bis 5 Gew.%, bezogen auf das Kulturmedium, angewendet.

Die für die Hydroxylierung verantwortlichen Enzyme des Mikroorganismus werden zweckmäßig durch Pyrazin induziert.

Die zur Induktion verwendete Verbindung kann entweder während der Umsetzung des Pyrazinderivats (Substrat) anwesend sein oder die Zufuhr dieser Verbindung wird während der Umsetzung unterbunden. Vorzugsweise wird die Zufuhr der zur Induktion verwendeten Verbindungen während der Umsetzung des Pyrazinderivats entweder durch Stoppen der Zufuhr oder durch Zentrifugieren der Zellen unterbunden.

Vor der Substratzugabe werden die Zellen bis zu einer optischen Dichte von 100 bei 650 nm, vorzugsweise bis zu einer optischen Dichte von 10 bis 60 bei 650 nm angezogen (kultiviert).

Als Nährmedium für die Mikroorganismen können sowohl für die Kultivierung als auch für das eigentliche Verfahren die in der Fachwelt üblichen Medien angewendet werden. Vorzugsweise wird das Medium angewendet, dessen Zusammensetzung in Tabelle 1 angegeben ist.
Üblicherweise wird dann das eigentliche Verfahren mit ruhenden Zellen durchgeführt.

Das Pyrazinderivat der allgemeinen Formel I als Substrat kann einmalig oder kontinuierlich zur Zellsuspension zugeführt werden, vorzugsweise so, dass die Substrat-Konzentration im Kulturmedium 20% (w/v) nicht übersteigt.
Insbesondere übersteigt die Substrat-Konzentration nicht 5% (w/v) im Kulturmedium.

Die Umsetzung wird zweckmässig in einem pH-Bereich von 4 bis 10, vorzugsweise von 6 bis 8 durchgeführt.

Üblicherweise wird die Umsetzung bei einer Temperatur von 0 bis 55 °C, vorzugsweise bei 20 bis 40 °C, durchgeführt.

Nach einer üblichen Umsetzungsdauer von 5 bis 100 Stunden können die hydroxylierten Pyrazinderivate auf bekannte Weise isoliert werden, beispielsweise durch Extraktion mit einem geeigneten organischen Lösungsmittel. Zweckmässig werden die hydroxylierten Pyrazinderivate durch Extraktion mit chlorierten organischen Lösungsmitteln, wie beispielsweise chlorierten Kohlenwasserstoffen oder Ethylacetat, isoliert.

Beispiel 1

Isolierung von Pyrazin-metabolisierenden Mikroorganismen

Aerobe Pyrazin-metabolisierende Mikroorganismen wurden in A + N-Medium (Tabelle 1) mit Zusatz von 0,1% (w/v) Pyrazin als einziger Kohlenstoff- und Energiequelle angereichert. Die allgemeinen Techniken zur Isolation von Mikroorganismen sind beispielsweise in "G.Drews, Mikrobiologisches Praktikum, 4.Aufl., Springer Verlag, 1983" beschrieben.
Als Inokulum wurden Proben aus dem Erdreich, Kläranlagen, Kompost und Ameisenhaufen verwendet. Die Anreicherungen wurden in Schüttelkolben bei 30 °C angezogen. Nach dreimaligem Überimpfen in frisches

Medium wurden die Anreicherungen auf dem gleichen Medium mit Zusatz von 16 g Agar pro Liter ausgestrichen und bei 30 °C inkubiert. Nach mehrmaligem Ausstreichen auf Agarmedium konnten Reinkulturen isoliert werden.

Tabelle 1: A+N-Medium

| Zusammensetzung: | Konzentration (mg/l) |
|---|---|
| $(NH_4)_2SO_4$ | 2000 |
| $Na_2HPO_4$ | 2000 |
| $KH_2PO_4$ | 1000 |
| NaCl | 3000 |
| $MgCl_2 \cdot 6H_2O$ | 400 |
| $CaCl_2 \cdot 2H_2O$ | 14,5 |
| $FeCl_3 \cdot 6H_2O$ | 0,8 |
| Pyridoxal-Hydrochlorid | $10 \cdot 10^{-3}$ |
| Riboflavin | $5 \cdot 10^{-3}$ |
| Nicotinsäureamid | $5 \cdot 10^{-3}$ |
| Thiamin-Hydrochlorid | $2 \cdot 10^{-3}$ |
| Biotin | $2 \cdot 10^{-3}$ |
| Pantothensäure | $5 \cdot 10^{-3}$ |
| p-Aminobenzoat | $5 \cdot 10^{-3}$ |
| Folsäure | $2 \cdot 10^{-3}$ |
| Vitamin B12 | $5 \cdot 10^{-3}$ |
| $ZnSO_4 \cdot 7H_2O$ | $100 \cdot 10^{-3}$ |
| $MnCl_2 \cdot 4H_2O$ | $90 \cdot 10^{-3}$ |
| $H_3BO_3$ | $300 \cdot 10^{-3}$ |
| $CoCl_2 \cdot 6H_2O$ | $200 \cdot 10^{-3}$ |
| $CuCl_2 \cdot 2H_2O$ | $10 \cdot 10^{-3}$ |
| $NiCl_2 \cdot 6H_2O$ | $20 \cdot 10^{-3}$ |
| $Na_2MoO_4 \cdot 2H_2O$ | $30 \cdot 10^{-3}$ |
| $EDTANa_2 \cdot 2H_2O$ | $5 \cdot 10^{-3}$ |
| $FeSO_4 \cdot 7H_2O$ | $2 \cdot 10^{-3}$ |

(pH der Lösung wurde auf 7.0 eingestellt)

Beispiel 2

Umsetzung von 3-Chlorpyrazin zu 3-Chlor-2-hydroxypyrazin Agrobacterium sp.

(DSM-Nr. 6136) wurde im A + N-Medium mit 0,1% (w/v) Pyrazin in einem Fermenter bei pH 7 und einer Temperatur von 30 °C angezogen. Anschliessend wurden die Zellen zentrifugiert, im A + N-Medium wieder resuspendiert und eine optische Dichte bei 650 nm von 10 eingestellt. Diese Zellsuspension wurde in einen Schüttelkolben gegeben und mit 26 mmol 3-Chlorpyrazin pro Liter (0,3% w/v) versetzt.
Nach einer Inkubation bei 30 °C von 8 h auf einer Schüttelmaschine wurden 20 mmol 3-Chlor-2-hydroxypyrazin pro Liter, entsprechend einer Ausbeute von 77%, nachgewiesen.

Die Beispiele 3 bis 5 wurden entsprechend zu Beispiel 2 durchgeführt und sind in Tabelle 2 zusammengefasst.

Die Stellung der Hydroxylgruppe wurde gemäss den Angaben von Mac Donald JC, Bishop GG, Mazurek (1976), Tetrahedron 32, S.655ff., bestimmt.

Tabelle 2

| Beispiel | Substrat | Konz.des Heterocyclus in % (w/v) im Medium | Reakt. zeit in h | Endprodukt | Ausbeute in % |
|---|---|---|---|---|---|
| 3 | 2-Methylpyrazin | 0,2 | 1 | 2-Hydroxy-6-methylpyrazin | 50 |
| 4 | 2-Ethylpyrazin | 0,2 | 24 | 6-Ethyl-2-hydroxypyrazin | 20 |
| 5 | 2,3-Dimethylpyrazin | 0,2 | 10 | 2-Hydroxy-5,6-dimethylpyrazin | 20 |

## Patentansprüche

1. Mikroorganismen, die befähigt sind, mit Pyrazin als einziger Kohlenstoff-, Stickstoff- und Energiequelle zu wachsen, und als Substrat Pyrazinderivate der allgemeinen Formel

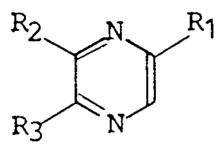

I

worin $R_1$ ein Wasserstoffatom oder ein Halogenatom bedeutet und $R_2$ und $R_3$ gleich oder verschieden sind und ein Wasserstoffatom oder eine $C_1$-$C_4$-Alkylgruppebedeuten, aber $R_1$, $R_2$ und $R_3$ nicht gleichzeitig Wasserstoff sind, in ein hydroxyliertes Pyrazinderivat der allgemeinen Formel

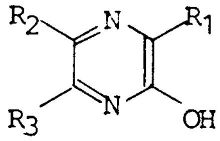

II

worin $R_1$, $R_2$ und $R_3$ die oben genannte Bedeutung haben, umzusetzen, wobei letzteres im Wachstumsmedium akkumuliert wird.

2. Mikroorganismus nach Patentanspruch 1 mit der Bezeichnung Agrobacterium sp. hinterlegt bei der DSM mit der Nummer 6136 und dessen Deszendenten und Mutanten.

3. Verfahren zur Herstellung von hydroxylierten Pyrazinderivaten, dadurch gekennzeichnet, dass man mit den Mikroorganismen gemäss einem der Patentansprüche 1 oder 2 ein Pyrazinderivat der allgemeinen Formel

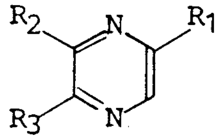

I

worin $R_1$ ein Wasserstoffatom oder ein Halogenatom bedeutet und $R_2$ und $R_3$ gleich oder verschieden sind und ein Wasserstoffatom oder eine $C_1$-$C_4$-Alkylgruppe bedeuten, aber $R_1$, $R_2$ und $R_3$ nicht gleichzeitig Wasserstoff sind, in ein hydroxyliertes Pyrazinderivat der allgemeinen Formel

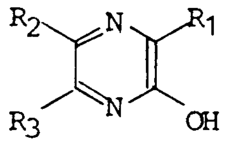

II

worin $R_1$, $R_2$ und $R_3$ die oben genannte Bedeutung haben, überführt und das angereicherte Produkt isoliert.

4. Verfahren nach Patentanspruch 3, dadurch gekennzeichnet, dass die wirksamen Enzyme des Mikroorganismus mit Pyrazin induziert werden.

5. Verfahren nach mindestens einem der Patentansprüche 3 oder 4, dadurch gekennzeichnet, dass die Umsetzung unter einmaliger oder kontinuierlicher Substrat-Zugabe durchgeführt wird, so dass die Substrat-Konzentration im Kulturmedium 20% (w/v) nicht übersteigt.

6. Verfahren nach mindestens einem der Patentansprüche 3 bis 5, dadurch gekennzeichnet, dass die Umsetzung bei einem pH von 4 bis 10 durchgeführt wird.

7. Verfahren nach mindestens einem der Patentansprüche 3 bis 6, dadurch gekennzeichnet, dass die Umsetzung bei Temperaturen von 0 bis 55 °C durchgeführt wird.

8. 6-Ethyl-2-hydroxypyrazin.